(19)

**Europäisches Patentamt**

**European Patent Office**

**Office européen des brevets**

(11)  **EP 4 730 236 A1**

(12)  # EUROPEAN PATENT APPLICATION

(43) Date of publication:
**22.04.2026 Bulletin 2026/17**

(21) Application number: **24206857.5**

(22) Date of filing: **16.10.2024**

(51) International Patent Classification (IPC):
**G06Q 10/0639** (2023.01)  **G05B 23/02** (2006.01)
**G06N 20/00** (2019.01)  **G16C 20/70** (2019.01)

(52) Cooperative Patent Classification (CPC):
**G06Q 10/06393; G05B 23/0283; G06N 20/00;
G06N 20/10; G16C 20/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **SABIC Global Technologies B.V.
4612 PX Bergen op Zoom (NL)**

(72) Inventors:
- **Abu Al-Hamayel, Saad Sameer
  6160 GA Geleen (NL)**
- **Kumar, Praveen
  6160 GA Geleen (NL)**

(74) Representative: **Sabic INDIA Intellectual Property
Group
P.O. Box 3008
6160 GA Geleen (NL)**

(54) **DYNAMIC CATALYST LIFECYCLE MODEL**

(57)     A dynamic catalyst lifecycle model is described. In some embodiments, real-time external data associated with a catalyst is captured. Actual operational data associated with a petrochemical plant is obtained and the catalyst is used in chemical processes at the petrochemical plant. A selectivity of the catalyst is continuously predicted under different loads and in real-time using a trained machine learning model. The actual operational data and the real-time external data are used as inputs to the trained machine learning model and the trained machine learning model is trained using an actual selectivity and historical catalyst performance.

EP 4 730 236 A1

# EP 4 730 236 A1

**Description**

<u>Technical Field</u>

This disclosure relates generally to modeling an optimum lifecycle of a catalyst.

<u>Background</u>

Chemical processes are utilized at petrochemical plants during production and treatment of hydrocarbons to obtain various products. Such processes are carried out in the presence of a catalyst to obtain a desired reaction. The reactivity, selectivity, and performance of a catalyst may vary and degrade over time.

<u>Summary</u>

An embodiment described herein provides a system, including one or more memory modules and one or more hardware processors communicably coupled to the one or more memory modules. The one or more hardware processors are configured to execute instructions stored on the one or more memory modules to perform operations including capturing real-time external data associated with a catalyst; obtaining actual operational data associated with a petrochemical plant, wherein the catalyst is used in chemical processes at the petrochemical plant; and continuously predicting a selectivity of the catalyst under different loads and in real-time using a trained machine learning model, wherein the actual operational data, the real-time external data, are inputs to the trained machine learning model, and wherein the trained machine learning model is trained using an actual selectivity and historical catalyst performance.

An embodiment described herein provides a method, including capturing, using at least one hardware processor, real-time external data associated with a catalyst; obtaining, using the at least one hardware processor, actual operational data associated with a petrochemical plant, wherein the catalyst is used in chemical processes at the petrochemical plant; and continuously predicting, using the at least one hardware processor, a selectivity of the catalyst under different loads and in real-time using a trained machine learning model, wherein the actual operational data, the real-time external data, are inputs to the trained machine learning model, and wherein the trained machine learning model is trained using an actual selectivity and historical catalyst performance.

An embodiment described herein provides an apparatus including a non-transitory, computer readable, storage medium that stores instructions that, when executed by at least one processor, cause the at least one processor to perform operations including: capturing real-time external data associated with a catalyst; obtaining actual operational data associated with a petrochemical plant, wherein the catalyst is used in chemical processes at the petrochemical plant; and continuously predicting a selectivity of the catalyst under different loads and in real-time using a trained machine learning model, wherein the actual operational data, the real-time external data, are inputs to the trained machine learning model, and wherein the trained machine learning model is trained using an actual selectivity and historical catalyst performance.

In some embodiments, an optimum time to replace the catalyst at the petrochemical plant is determined using the predicted selectivity.

In some embodiments, the optimum time to replace the catalyst at the petrochemical plant is determined using a physics-based model for the catalyst.

In some embodiments, the catalyst is replaced at the petrochemical plant at the optimum time by having a continuous technical-economical evaluation.

In some embodiments, the different loads comprise a minimum load, a current load, and a maximum load.

In some embodiments, continuously predicting the selectivity of the catalyst under different includes inputting varying load data to the trained machine learning model.

In some embodiments, the predicted selectivity, the actual operational data, the real-time external data, the actual selectivity, and the historical catalyst performance are rendered on a dashboard in real-time.

In some embodiments, the real-time external data are costs associated with acquisition of the catalyst.

In some embodiments, the selectivity is predicted to an end-of-run for the catalyst.

In some embodiments, the catalyst is an ethylene oxide catalyst in a petrochemical plant that produces ethylene oxide.

<u>Brief Description of Drawings</u>

Figure 1A shows a catalyst lifecycle.
Figure 1B is a process flow diagram of a process that enables a dynamic catalyst lifecycle model.

Figure 2 shows a system including real-time data, dynamic catalyst lifecycle model and a catalyst selectivity model.
Figure 3A is a first visualization of the output of a trained machine learning model.
Figure 3B is a second visualization of the output of a trained machine learning model.
Figure 4 is a schematic illustration of an example controller (or control system) for that enables a dynamic catalyst lifecycle model.

Detailed Description

Catalyst replacement is driven by the performance of the catalyst and economic considerations associated with the catalyst. The replacement requires a shutdown of the petrochemical plant, which can be costly and time consuming, however fresh catalyst consumes less raw material when compared with an old, used catalyst. Traditionally, the performance of the catalyst and economic considerations associated with the catalyst are limited to infrequent assessments, typically once or twice throughout the catalyst life. However operation conditions, market prices, and raw material costs are dynamic and vary throughout the catalyst lifecycle.

The present techniques enable a dynamic catalyst lifecycle model. Catalysts, such as an ethylene oxide catalyst, are used for producing ethylene glycols. Costs associated with acquiring raw materials and market prices of the catalysts vary widely within short periods of time. Real-time external data, such as changes in raw material costs and market prices, are continuously evaluated with actual operational data associated with a petrochemical plant to predict an end of run selectivity of the catalyst. An optimum time to replace the catalyst is determined based on, at least in part, the predicted selectivity of the catalyst as well as the best economical cycle.

Some of the advantages of these techniques include an accurate prediction of catalyst selectivity based on actual operational conditions, plant load, and actual selectivity. Further, the present techniques determine the impact of changes in market prices on an optimal operation period for the catalyst. The predicted selectivity is used to continuously update the catalyst lifecycle period to minimize losses and plant downtime associated with catalyst replacement. Further, the present techniques enable proper planning of maintenance turnaround time and forecast end of cycle selectivity.

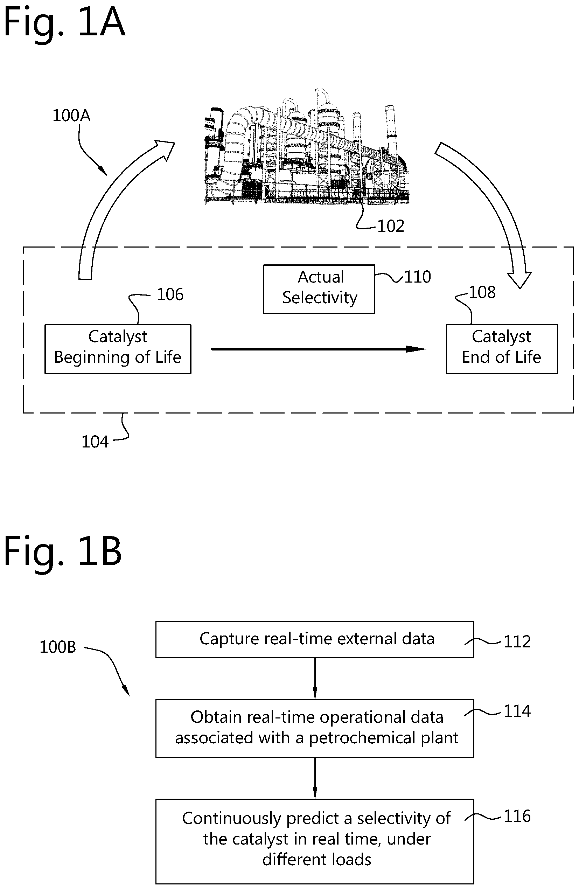

Figure 1A shows an environment 100A including a petrochemical plant shown at reference number 102 (petrochemical plant 102) and associated catalyst shown at reference number 104 (catalyst 104). In examples, the petrochemical plant 102 is petrochemical plant such as an olefins plant. For example, olefins are produced by operations at the petrochemical plant 102 using the catalyst 104 during a run, such as plant operations. A lifecycle of the catalyst 104 is shown by the catalyst beginning of life 106, the catalyst end of life 108, and actual selectivity 110 of the catalyst.

Catalysts, such as catalyst 104 (e.g., ethylene oxide catalyst) lose selectivity over time. Additionally, as selectivity decreases reactions using the catalyst consume more raw material (e.g., such as ethylene). For purposes of description, the catalyst 104 is described as a silver-based catalyst used to promote oxidation of ethylene to produce ethylene oxide. However, any catalyst can be used according to the present techniques. For example, the catalyst may be a palladium-based catalyst, such as a catalyst for a butane unit in a petrochemical plant that produces olefins, which is, typically changed every 10 years. The selectivity for palladium-based catalysts reduce over time, and the palladium-based catalysts are eventually replaced and the materials in the spent catalysts are recovered.

In examples, the selectivity of a catalyst refers to a yield of a reaction where the catalyst is used. Catalyst selectivity represents the ability of a catalyst to promote favorable reaction pathways that maximize the yield of desired products and minimize undesirable by-products. At some point, the catalyst is no longer effective for its intended purpose and is replaced with a fresh catalyst. In examples, catalyst selectively is reduced over time due to high temperatures and impurity generation. Traditionally, at the beginning of each catalyst cycle, calculations are conducted to determine a lifecycle period that achieves the most efficient use of the catalyst. Traditionally, the calculated lifecycle is static and does not change. Runs (e.g., plant operations including catalyst reactions) continue in accordance with the lifecycle period calculated at the beginning of the catalyst lifecycle. The calculations do not consider an actual selectivity of the catalyst, such as actual selectivity 110 that occurs in real-time at petrochemical plant 102.

Figure 1B is a process flow diagram of a process 100B that enables a dynamic catalyst lifecycle model. The present techniques model a lifecycle associated with a catalyst (e.g., catalyst 104 of Figure 1A), in real-time. In some embodiments, the model is continuously updated, in real-time. Accordingly, in some embodiments, the generated model varies in real-time. In some embodiments, the model is a trained machine learning model that obtains real-time external data and real-time operational data as input, and outputs a predicted selectivity of the catalyst.

In some embodiments, the dynamic lifecycle model of a catalyst, such as an ethylene oxide catalyst, enables an updated visibility into plant operations and maximizes the remaining useful life of the catalyst in real-time. The useful life refers to a time period ending with a "change out point" of the catalyst, where the favorable reaction pathways that maximize the yield of desired products as promoted by the catalyst is greater than the undesirable by-products of reactions. In examples, the change out point occurs at a point in time where changing the existing catalyst to a fresh catalyst is more profitable and/or less costly than continuing a run with the existing catalyst.

At block 112, real-time external data associated with a catalyst is captured. In examples, the real-time external data associated with a catalyst includes costs of raw materials associated with the catalyst and market prices of catalyst components and/or the purchase of the catalyst. For example, if the catalyst is an ethylene oxide catalyst used to produce ethylene oxide and eventually is used to produce ethylene glycol from ethylene, the catalyst may include raw materials such as silver, rhenium, and cesium. Costs associated with such raw materials, including oxygen, can vary continuously, in real-time as well as product prices (e.g., ethylene glycol). Further, the costs and market prices are dependent upon the interaction of demand and supply. In some embodiments, the costs or market prices are obtained from a database, such as a multi-model database that stores data in memory instead of storing data on a disk. In examples, the data is retrieved from a column-oriented in-memory database design that enables advanced analytics and high-speed transactions. Accordingly, the database stores and processes large amounts of data with low latency. In some embodiments, the costs or market prices are obtained from a third party and stored in the database.

At block 114, real-time operational data associated with a petrochemical plant (e.g., petrochemical plant 102 of Figure 1A) is obtained. In some embodiments, the catalyst is used in chemical processes at the petrochemical plant. In some embodiments, plant operational data includes operational costs, plant capacity utilization, maintenance costs, utilities costs, and the like. In some embodiments, the operational data is generated by various petrochemical plant operations, in real-time. In examples, real-time operational data is obtained by the model from an organizational data lake. In examples, the data lake is a centralized repository that ingests, stores, and enables processing of large volumes of data in its original form. The data lake can accommodate varying types of data. In some embodiments, the real-time external data and the real-time operational data is used to update the dynamic model of the catalyst lifecycle in real-time.

At block 116, a selectivity of the catalyst is predicted under different loads and in real-time based on the real-time operational data, the real-time external data, an actual selectivity, and historical catalyst performance. In some embodiments, the different loads comprise a minimum load, a current load, and a maximum load. In examples, a load refers to an operating capacity of a plant during a run. For example, load refers to a percentage of actual production at the plant as compared to the name plate (design) production, or the design capacity. For example, a plant nameplate capacity is 1000 MT/day (e.g., design capacity). If the actual production is 1000 MT/day, then the plant is operating at 100% plant load. If the actual production is 900 MT/day, then the plant is operating at 90% plant load. The plant load is proportional to the consumption of raw material. For example, operating at a higher load results in faster degradation of the catalyst. Continuously predicting the selectivity of the catalyst under different loads comprises inputting varying load data to a trained machine learning model. In some embodiments, the selectivity of the catalyst is output as a catalyst selectivity forecast over time.

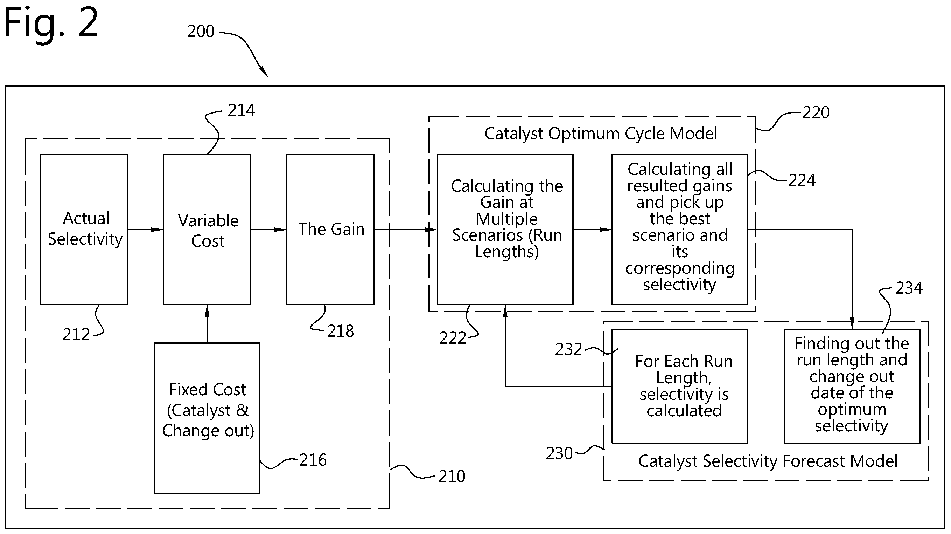

Figure 2 shows a system 200 including an optimum cycle model 220 and a catalyst selectivity model 230. In some embodiments, the process 100B of Figure 1B is implemented using the system 200 of Figure 2. Real-time data 210 includes real-time external data and real-time operational data. As shown in the example of Figure 2, an actual selectivity 212, variable costs 214, fixed costs 216, and a gain 218 are obtained in real-time. In examples, the real-time data 210 is updated continuously. For example, real-time external data is updated based on fluctuations in costs and market prices over time. Additionally, real-time operational data is updated responsive to plant operations over time.

In some embodiments, the actual selectivity drives the variable costs associated with plant operations using a catalyst. As shown in Figure 2, the variable costs 214 are a function of the actual selectivity 212 and fixed costs 216. In examples, fixed costs include utility and steam costs that are independent of the running status if the plant. As the actual selectivity 212 of the catalyst varies over one or more runs, variable costs 214 also change. For example, changes in the actual selectivity 212 can cause changes in the raw material consumed, which increases the cost of raw materials for a run. Changes in raw material consumed also changes utilities flows of the plant, which increases the costs of utilities.

A gain 218 is determined based on the variable cost. In some embodiments, the gain is a measure of an impact of changing a catalyst to a fresh catalyst over a period of time. For example, the gain quantifies an efficiency of a current catalyst when compared to an efficiency of a fresh catalyst. In some embodiments, the gain represents a profit over a period of time that arises due to a difference between the actual selectivity of a current catalyst and a predicted selectivity of a fresh catalyst. A fresh catalyst is an unused catalyst associated with a full, undegraded lifecycle. In examples, a guaranteed selectivity is provided by the vendor of the catalyst and is used as the predicted catalyst selectivity of a new, fresh catalyst. For example, the vendor (e.g., a producer of the catalyst) determines information associated with the catalyst through petrochemical analysis and experimentation. The guaranteed selectivity of the fresh catalyst represents an actual selectivity associated with the catalyst at the beginning of life of the catalyst.

A catalyst optimum cycle model 220 is executed to visualize and compare selectivity for multiple scenarios using real-time data 210 and a forecast selectivity output by a catalyst selectivity forecast model 230. In some embodiments, a scenario refers to a set of parameters associated with a future run using the current catalyst. In examples, the set of parameters includes predicted external data and predicted operation data for a time period associated with the future run, where the future run ends at a change out point for the catalyst. A scenario includes predicted external data and predicted operation data for a series of days, months, or years in the future. For example, a first scenario corresponds to a run length from a current state to 24 months into the future; a second scenario corresponds to a run length from a current state to 26 months into the future; a third scenario corresponds to a run length from a current state to 28 months into the future; and so on. At block 222, a gain is predicted for multiple scenarios with varying run lengths based on real-time data 210 and feedback from a catalyst selectivity forecast model 230. Gain is iteratively predicted at block 222 responsive to updates or changes in the real-time data 210. For example, changes in the actual selectivity 212 ultimately cause changes in the predicted gain for the multiple scenarios with varying run lengths. At block 222, the predicted gain is instantly updated based on, at least in part, changes in the actual selectivity.

At block 224, the predicted gains are compared to select an optimum scenario from the multiple scenarios at each iteration. Each of the multiple scenarios is associated with a predicted end of life selectivity corresponding to the end of a respective run. The optimum scenario is the scenario with a highest gain over time when compared to other scenarios. A cycle factor is applied to each of the multiple scenarios to enable a comparison of the multiple scenarios. For each iteration, the selected optimum scenario is obtained by the catalyst selectivity forecast model 230. The catalyst selectivity forecast model 230 obtains the run length, end of run selectivity, and change out point associated with the selected optimum scenario at block 234. Based on the change out point, a selectivity is calculated over the entire run for the optimum scenario at block 232. The calculated selectivity for the optimum scenario is fed back to the optimum cycle model 220.

In some embodiments a dynamic catalyst lifecycle model includes the catalyst optimum cycle model and catalyst selectivity forecast model. The catalyst optimum cycle model visualizes multiple scenarios, and determines an optimum scenario and corresponding end of run selectivity. As time progresses, the optimum scenario and corresponding selectivity updated based on the real-time data and gains calculated at multiple scenarios. The updated optimum scenario and corresponding selectivity are iteratively evaluated by the catalyst selectivity forecast model. In some embodiments, the selectivity output by the catalyst selectivity forecast model 230 is generated using a machine learning model trained using data associated with an actual selectivity and historical catalyst performance. In examples, historical catalyst performance includes, for example, real-time data 210, optimum selectivity, and catalyst optimum run length.

In examples, a selectivity is calculated based on a proposed change out date (or a proposed catalyst cycle run length) at block 232. In examples, a cycle factor is calculated as follows:

$$\frac{1}{(Online\ Days + Change\ Out\ Days)}$$

Online days refer to a number of days where the plant is operating according to its intended purpose. Change out days refer to a number of days where the plant is not operating according to intended purpose due to changing of the catalyst. Real-time data, such as variable costs, products prices, catalyst prices, planned production rate, are calculated based on the online days and change out days. For example, the real-time data associated with a particular run is weighted by the cycle factor to determine net gain for a respective scenario. The cycle factor normalizes each respective scenario to a same or similar scale so the associated selectivity can be compared across the multiple scenarios at which each day in different scenarios can be compared together.

The daily net gain is calculated as follows:

$$[Abs(Gain - Fixed\ Cost) * Cycle\ Factor]$$

A selectivity is calculated for each day of a respective scenario by dividing the produced desired production (e.g., Ethylene Oxide) over total material produced from the reactions. In examples, the optimum selectivity is the selectivity corresponding to the optimum change out time based on net gain, which is a result of comparing all run length scenarios and choosing the best one of them. If gain associated with a respective run is higher than cost, then this indicates that a changeover should be delayed. If cost is higher than gain, then this indicates that the plant will incur loss if changeover is delayed.

In examples, a catalyst run length is calculated by determining optimum online days, which are the online days to reach optimum selectivity estimation as output by the catalyst selectivity forecasting model. The remaining days are determined by subtracting the current online days from the optimum online days as follows:

$$Remaining\ Days = Optimum\ Online\ Days - Current\ Online\ Days$$

In examples, the remaining days refer to the number of days left prior to a point in time where operating with the current catalyst causes an opportunity loss (e.g., loss of potential gain) compared with halting production to change to a fresh catalyst.

The catalyst change out point equals the time when maximum accumulated contribution margin for specified operation time at full load is reached. In examples, a contribution margin, revenue, variable costs, and depreciated catalyst costs are data associated with historical catalyst performance. In some embodiments, the contribution margin compared across scenarios, and the scenario having a highest contribution margin corresponds to the optimum scenario as described with respect to Figure 2. In examples, these values are determined as follows:

$$The\ Contribution\ Margin$$
$$= Revnue - Operating\ Cost$$
$$- Depreciated\ Catalysts'\ Cost\ over\ Operation\ Period$$

$$Revenue = Production\ in\ Full\ Load * Selling\ Price$$

$$Variable\ Cost = \sum (Raw\ Material\ Cost, Utility\ Cost)$$

$$Depreciated\ Catalyst\ Cost = \frac{\sum Catalyst\ Capex}{Period}$$

In an example, the following analysis is used to obtain values associated with historical catalyst performance. Assume a current online days of 100 days, and equivalent ethylene oxide (EOE) Production is 71.5 metric tons per hour (MT/Hr), Production percentages: mono-ethylene glycol (MEG) (88%), di-ethylene glycol (DEG) (11%), tri ethylene glycol (TEG) (1%). EOE refers to the amount of ethylene oxide that is required to produce certain amount of ethylene glycol. The production percentages represent the amount of each respective product relative to the total products produced. Each product is evaluated independently at a respective selling price. In this example, the gain (MM$/Day 0.96) is calculated as the revenue from product (MM$/Day 1.63) minus variable cost (MM$/Day 0.67). The gain is calculated for the 100th day, and the revenue from the generated product is calculated based on all product flows and their respective prices. Additionally, the variable cost is calculated based on raw material, utilities flows, and their respective prices.

First, a cumulative gain till the 100th day (MM$ 96.24) is calculated as the sum of daily gains from the 1st day to 100th day of a catalyst run when the catalyst is fresh. The cumulative gain from a fresh catalyst till current days online(MM$) minus the sum of current run gain is determined. Second, a cumulative potential gain (MM$ 95.71) for a next 100 days is determined as the sum of daily gains from 101st to a 200th day of a catalyst run. The cumulative potential gain from the old catalyst for the same online days (MM$) minus the sum of the current run gain if continuing with the same catalyst for next 100 online days is determined.

Third, assuming scenario of running the catalyst for 100 days only, a gain (MM$ 0.53) is determined as the cumulative gain till a 100th day of the catalyst run (MM$ 96.24) minus the cumulative potential gain (MM$ 95.71). The gain ($) represents the effective gain which is representation of extra gain in selectivity due to catalyst changeover whereas the potential gain is the gain if catalyst was not changed and kept running for an extra 100 days. Fourth, fixed cost (MM$ 21.98) are calculated as a catalyst cost (MM$ 19.98) plus a catalyst change out cost (MM$ 2). Fifth, a change out delta (MM$/Day 0.18) is calculated as the absolute value of (Gain (MM$ 0.53) minus the fixed cost (MM $ 21.98)) multiplied by the (1/(Current Online Days (Days 100) + Change out Period (Days 18))). A minimum of change out delta among all days will give optimum selectivity, where changeover needs to be done. Additionally, the number of online days to reach optimum selectivity is estimated through the catalyst selectivity forecast model 230.

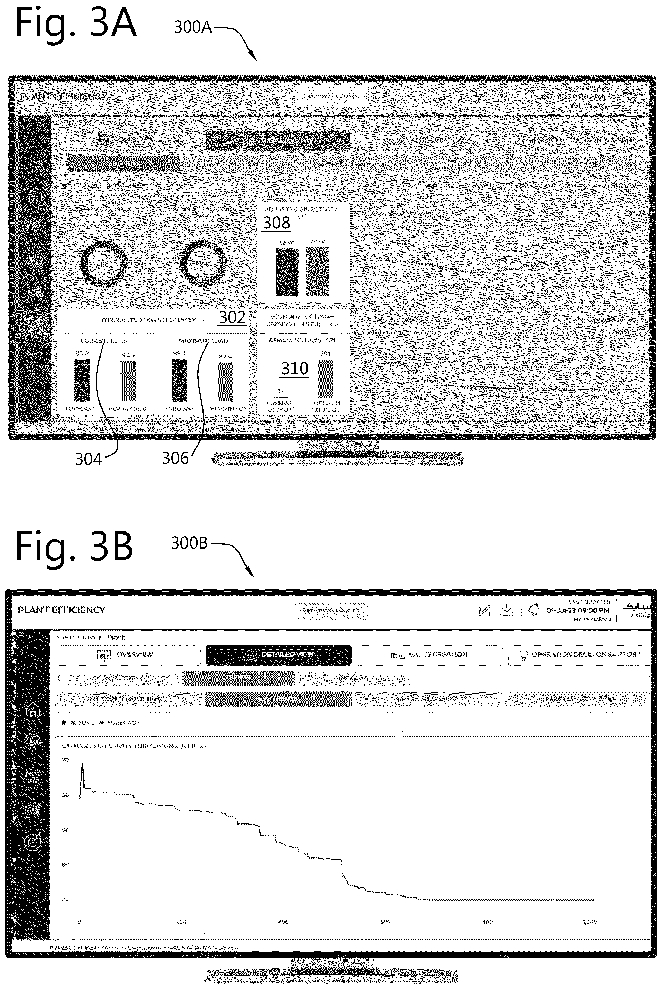

Accordingly, the present techniques enable a determination of the updated optimum change out point considering plant performance and conditions, continuously updated costs and prices, and a selectivity forecast at different scenarios. Traditional techniques fail to consider actual operational conditions and load, actual selectivity throughout the run, changes in raw material, and changes in market prices are not considered. The model uses the historical data for catalyst performance and different plant loads/conditions to forecast a drop in selectivity in two scenarios: (1) continuing at current load; and (2) increasing the load to maximum. Figure 3A is a first visualization 300A of the output of a trained machine learning model. A forecast end-of-run selectivity 302 is shown and includes forecast selectivity at the current load 304 and a maximum load 306. Adjusted selectivity 308 is the selectivity of the catalyst adjusted to the actual operation conditions at the plant in order to be comparable with the vendor information associated with the catalyst, determined through petrochemical analysis and experimentation. The economic optimum catalyst online 310 shows the optimum number of days remaining for the current catalyst. The model output shown in Figure 3A varies in real-time based on real-time external data associated with a respective catalyst and actual operational data associated with a petrochemical plant.

Figure 3B is a second visualization 300B of the output of a trained machine learning model. In the example of Figure 3B, a plot of catalyst selectivity over time is shown in decline. The model also identifies the optimum point in time to change out the catalyst. In examples, the plot shown in Figure 3B varies in real-time based on real-time external data associated with a respective catalyst and actual operational data associated with a petrochemical plant.

The present techniques develop a trained machine learning model by combining catalyst design data and statistical data to determine catalyst selectivity up to end of run at multiple load scenarios. In some embodiments, catalyst design data is a prediction of selectivity of the catalyst at every month of the catalyst run, including reaction temperature prediction and operation parameters. The predicted selectivity of the catalyst design data is calculated using proprietary information and experiment by the vendor of the catalyst. A visualization platform can visualize a selectivity forecast up to end of run and optimum change point for multiple scenarios. The model uses the historical data for catalyst performance and different plant loads/conditions to forecast the drop in selectivity in two scenarios: continuing at current load and increasing the load to maximum. Further, the model identifies the optimum point in time to change out the catalyst.

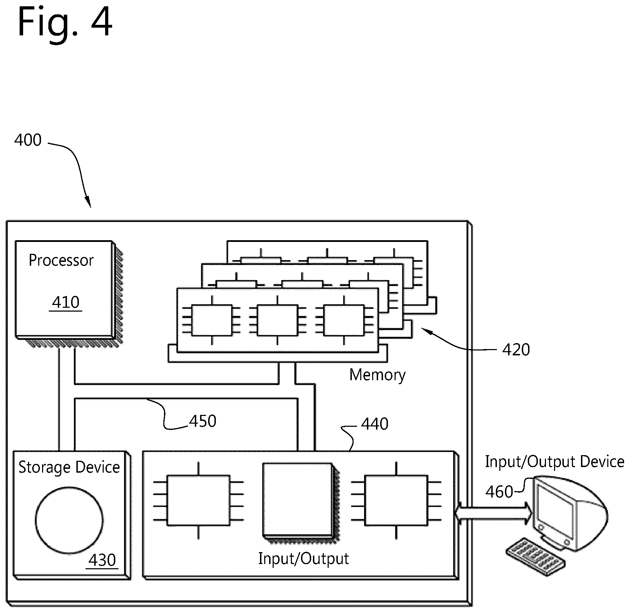

Figure 4 is a schematic illustration of an example controller 400 (or control system) that enables a dynamic catalyst lifecycle model. For example, the controller 400 may be operable according to the process 100 of Figure 1, and visualized as shown in Figure 3A and Figure 3B. The controller 400 is intended to include various forms of digital computers, such as printed circuit boards (PCB), processors, digital circuitry, or otherwise parts of a system for supply chain alert management. Additionally the system can include portable storage media, such as, Universal Serial Bus (USB) flash drives. For example, the USB flash drives may store operating systems and other applications. The USB flash drives can include input/output components, such as a wireless transmitter or USB connector that may be inserted into a USB port of another computing device.

The controller 400 includes a processor 410, a memory 420, a storage device 430, and an input/output interface 440 communicatively coupled with input/output devices 460 (for example, displays, keyboards, measurement devices, sensors, valves, pumps). Each of the components 410, 420, 430, and 440 are interconnected using a system bus 450. The processor 410 is capable of processing instructions for execution within the controller 400. The processor may be designed using any of a number of architectures. For example, the processor 410 may be a CISC (Complex Instruction Set Computers) processor, a RISC (Reduced Instruction Set Computer) processor, or a MISC (Minimal Instruction Set Computer) processor.

In one implementation, the processor 410 is a single-threaded processor. In another implementation, the processor 410 is a multi-threaded processor. The processor 410 is capable of processing instructions stored in the memory 420 or on the storage device 430 to display graphical information for a user interface on the input/output interface 440.

The memory 420 stores information within the controller 400. In one implementation, the memory 420 is a computer-readable medium. In one implementation, the memory 420 is a volatile memory unit. In another implementation, the memory 420 is a nonvolatile memory unit.

The storage device 430 is capable of providing mass storage for the controller 400. In one implementation, the storage device 430 is a computer-readable medium. In various different implementations, the storage device 430 may be a floppy disk device, a hard disk device, an optical disk device, or a tape device.

The input/output interface 440 provides input/output operations for the controller 400. In one implementation, the input/output devices 460 includes a keyboard and/or pointing device. In another implementation, the input/output devices 460 includes a display unit for displaying graphical user interfaces.

In some embodiments, the system 400 obtains real-time external data and continuously evaluates the real-time external data with actual operational data to predict an end of run selectivity of a catalyst. In some embodiments, the real-time external data and actual operational data are obtained from a multi-model database or an organizational data lake. In examples, the system 400 includes or is communicatively coupled with a multi-model database or an organizational data lake.

In some embodiments, the system 400 renders the predicted selectivity, the actual operational data, the real-time external data, the actual selectivity, and the historical catalyst performance on a dashboard in real-time for a number of different plants via the input/output devices 460. In examples, the first visualization 300A (Figure 3A), the second visualization 300B (Figure 3B), or any combinations thereof are rendered via the input/output devices 460. The renderings are updated in real-time to show the selectivity of the catalyst predicted under different loads and in real-time based on the actual operational data, the real-time external data, an actual selectivity, and historical catalyst performance.

There can be any number of controllers 400 associated with, or external to, a computer system containing controller 400, with each controller 400 communicating over a network. Further, the terms "client," "user," and other appropriate terminology can be used interchangeably, as appropriate, without departing from the scope of the present disclosure. Moreover, the present disclosure contemplates that many users can use one controller 400 and one user can use multiple controllers 400.

Implementations of the subject matter and the functional operations described in this specification can be implemented in digital electronic circuitry, in tangibly embodied computer software or firmware, in computer hardware, including the structures disclosed in this specification and their structural equivalents, or in combinations of one or more of them. Software implementations of the described subject matter can be implemented as one or more computer programs. Each computer program can include one or more modules of computer program instructions encoded on a tangible, non-transitory, computer-readable computer-storage medium for execution by, or to control the operation of, data processing apparatus. Alternatively, or additionally, the program instructions can be encoded in/on an artificially generated propagated signal. The example, the signal can be a machine-generated electrical, optical, or electromagnetic signal that is generated to encode information for transmission to suitable receiver apparatus for execution by a data processing apparatus. The computer-storage medium can be a machine-readable storage device, a machine-readable storage substrate, a random or serial access memory device, or a combination of computer-storage mediums.

The terms "data processing apparatus," "computer," and "electronic computer device" (or equivalent as understood by one of ordinary skill in the art) refer to data processing hardware. For example, a data processing apparatus can encompass all kinds of apparatus, devices, and machines for processing data, including by way of example, a programmable processor, a computer, or multiple processors or computers. The apparatus can also include special purpose logic circuitry including, for example, a central processing unit (CPU), a field programmable gate array (FPGA), or an application specific integrated circuit (ASIC). In some implementations, the data processing apparatus or special purpose logic circuitry (or a combination of the data processing apparatus or special purpose logic circuitry) can be hardware- or software-based (or a combination of both hardware- and software-based). The apparatus can optionally include code that creates an execution environment for computer programs, for example, code that constitutes processor firmware, a protocol stack, a database management system, an operating system, or a combination of execution environments. The present disclosure contemplates the use of data processing apparatuses with or without conventional operating systems, for example, LINUX, UNIX, WINDOWS, MAC OS, ANDROID, or IOS.

A computer program, which can also be referred to or described as a program, software, a software application, a module, a software module, a script, or code, can be written in any form of programming language. Programming languages can include, for example, compiled languages, interpreted languages, declarative languages, or procedural languages. Programs can be deployed in any form, including as stand-alone programs, modules, components, subroutines, or units for use in a computing environment. A computer program can, but need not, correspond to a file in a file system. A program can be stored in a portion of a file that holds other programs or data, for example, one or more scripts stored in a markup language document, in a single file dedicated to the program in question, or in multiple coordinated files storing one or more modules, sub programs, or portions of code. A computer program can be deployed for execution on one computer or on multiple computers that are located, for example, at one site or distributed across multiple sites that are interconnected by a communication network. While portions of the programs illustrated in the various figures may be shown as individual modules that implement the various features and functionality through various objects, methods, or processes, the programs can instead include a number of sub-modules, third-party services, components, and libraries. Conversely, the features and functionality of various components can be combined into single components as appropriate. Thresholds used to make computational determinations can be statically, dynamically, or both statically and dynamically determined.

The methods, processes, or logic flows described in this specification can be performed by one or more programmable computers executing one or more computer programs to perform functions by operating on input data and generating output. The methods, processes, or logic flows can also be performed by, and apparatus can also be implemented as, special purpose logic circuitry, for example, a CPU, an FPGA, or an ASIC.

Computers suitable for the execution of a computer program can be based on one or more of general and special purpose microprocessors and other kinds of CPUs. The elements of a computer are a CPU for performing or executing instructions and one or more memory devices for storing instructions and data. Generally, a CPU can receive instructions and data from (and write data to) a memory. A computer can also include, or be operatively coupled to, one or more mass storage devices for storing data. In some implementations, a computer can receive data from, and transfer data to, the mass storage devices including, for example, magnetic, magneto optical disks, or optical disks. Moreover, a computer can be embedded in another device, for example, a mobile telephone, a personal digital assistant (PDA), a mobile audio or video player, a game console, a global positioning system (GPS) receiver, or a portable storage device such as a universal serial bus (USB) flash drive.

Computer readable media (transitory or non-transitory, as appropriate) suitable for storing computer program instructions and data can include all forms of permanent/non-permanent and volatile/non-volatile memory, media, and memory devices. Computer readable media can include, for example, semiconductor memory devices such as random access memory (RAM), read only memory (ROM), phase change memory (PRAM), static random access memory (SRAM), dynamic random access memory (DRAM), erasable programmable read-only memory (EPROM), electrically erasable programmable read-only memory (EEPROM), and flash memory devices. Computer readable media can also include, for example, magnetic devices such as tape, cartridges, cassettes, and internal/removable disks. Computer readable media can also include magneto optical disks and optical memory devices and technologies including, for example, digital video disc (DVD), CD ROM, DVD+/-R, DVD-RAM, DVD-ROM, HD-DVD, and BLURAY. The memory can store various objects or data, including caches, classes, frameworks, applications, modules, backup data, jobs, web pages, web page templates, data structures, database tables, repositories, and dynamic information. Types of objects and data stored in memory can include parameters, variables, algorithms, instructions, rules, constraints, and references.

Additionally, the memory can include logs, policies, security or access data, and reporting files. The processor and the memory can be supplemented by, or incorporated in, special purpose logic circuitry.

Implementations of the subject matter described in the present disclosure can be implemented on a computer having a display device for providing interaction with a user, including displaying information to (and receiving input from) the user. Types of display devices can include, for example, a cathode ray tube (CRT), a liquid crystal display (LCD), a light-emitting diode (LED), and a plasma monitor. Display devices can include a keyboard and pointing devices including, for example, a mouse, a trackball, or a trackpad. User input can also be provided to the computer through the use of a touchscreen, such as a tablet computer surface with pressure sensitivity or a multi-touch screen using capacitive or electric sensing. Other kinds of devices can be used to provide for interaction with a user, including to receive user feedback including, for example, sensory feedback including visual feedback, auditory feedback, or tactile feedback. Input from the user can be received in the form of acoustic, speech, or tactile input. In addition, a computer can interact with a user by sending documents to, and receiving documents from, a device that is used by the user. For example, the computer can send web pages to a web browser on a user's client device in response to requests received from the web browser.

The term "graphical user interface," or "GUI," can be used in the singular or the plural to describe one or more graphical user interfaces and each of the displays of a particular graphical user interface. Therefore, a GUI can represent any graphical user interface, including, but not limited to, a web browser, a touch screen, or a command line interface (CLI) that processes information and efficiently presents the information results to the user. In general, a GUI can include a plurality of user interface (UI) elements, some or all associated with a web browser, such as interactive fields, pull-down lists, and buttons. These and other UI elements can be related to or represent the functions of the web browser.

Implementations of the subject matter described in this specification can be implemented in a computing system that includes a back end component, for example, as a data server, or that includes a middleware component, for example, an application server. Moreover, the computing system can include a front-end component, for example, a client computer having one or both of a graphical user interface or a Web browser through which a user can interact with the computer. The components of the system can be interconnected by any form or medium of wireline or wireless digital data communication (or a combination of data communication) in a communication network. Examples of communication networks include a local area network (LAN), a radio access network (RAN), a metropolitan area network (MAN), a wide area network (WAN), Worldwide Interoperability for Microwave Access (WIMAX), a wireless local area network (WLAN) (for example, using 802.11 a/b/g/n or 802.20 or a combination of protocols), all or a portion of the Internet, or any other communication system or systems at one or more locations (or a combination of communication networks). The network can communicate with, for example, Internet Protocol (IP) packets, frame relay frames, asynchronous transfer mode (ATM) cells, voice, video, data, or a combination of communication types between network addresses.

The computing system can include clients and servers. A client and server can generally be remote from each other and can typically interact through a communication network. The relationship of client and server can arise by virtue of computer programs running on the respective computers and having a client-server relationship. Cluster file systems can be any file system type accessible from multiple servers for read and update. Locking or consistency tracking may not be necessary since the locking of exchange file system can be done at application layer. Furthermore, Unicode data files can be different from non-Unicode data files.

While this specification contains many specific implementation details, these should not be construed as limitations on the scope of what may be claimed, but rather as descriptions of features that may be specific to particular implementations. Certain features that are described in this specification in the context of separate implementations can also be implemented, in combination, in a single implementation. Conversely, various features that are described in the context of a single implementation can also be implemented in multiple implementations, separately, or in any suitable sub-combination. Moreover, although previously described features may be described as acting in certain combinations and even initially claimed as such, one or more features from a claimed combination can, in some cases, be excised from the combination, and the claimed combination may be directed to a sub-combination or variation of a sub-combination.

Furthermore, any claimed implementation is considered to be applicable to at least a computer-implemented method; a non-transitory, computer-readable medium storing computer-readable instructions to perform the computer-implemented method; and a computer system comprising a computer memory interoperably coupled with a hardware processor configured to perform the computer-implemented method or the instructions stored on the non-transitory, computer-readable medium.

Particular embodiments of the subject matter have been described. Other embodiments are within the scope of the following claims. For example, the actions recited in the claims can be performed in a different order and still achieve desirable results. As one example, some processes depicted in the accompanying figures do not necessarily require the particular order shown, or sequential order, to achieve desirable results.

**Claims**

1. A system, comprising:

   one or more memory modules;
   one or more hardware processors communicably coupled to the one or more memory modules, the one or more hardware processors configured to execute instructions stored on the one or more memory modules to perform operations comprising:

   capturing real-time external data associated with a catalyst;
   obtaining real-time operational data associated with a petrochemical plant, wherein the catalyst is used in chemical processes at the petrochemical plant; and
   continuously predicting a selectivity of the catalyst under different loads and in real-time using a trained machine learning model, wherein the actual operational data and the real-time external data are inputs to the trained machine learning model, and wherein the trained machine learning model is trained using an actual selectivity and historical catalyst performance.

2. The system of claim 1, wherein the operations comprise determining an optimum time to replace the catalyst at the petrochemical plant using the predicted selectivity.

3. The system of claim 2, wherein the optimum time to replace the catalyst at the petrochemical plant is determined using a physics-based model for the catalyst.

4. The system of claim 2, comprising replacing the catalyst at the petrochemical plant at the optimum time.

5. The system of any preceding claim, wherein the different loads comprise a minimum load, a current load, and a maximum load.

6. The system of claim any preceding claim, wherein continuously predicting the selectivity of the catalyst under different comprises inputting varying load data to the trained machine learning model.

7. The system of claim any preceding claim, comprising rendering the predicted selectivity, the actual operational data, the real-time external data, the actual selectivity, and the historical catalyst performance on a dashboard in real-time.

8. The system of claim any preceding claim, wherein the real-time external data are costs associated with acquisition of the catalyst.

9. The system of claim any preceding claim, wherein the selectivity is predicted to an end-of-run for the catalyst.

10. The system of claim any preceding claim, wherein the catalyst is an ethylene oxide catalyst in a petrochemical plant that produces ethylene oxide.

11. A method, comprising:

    capturing real-time external data associated with a catalyst;
    obtaining real-time operational data associated with a petrochemical plant, wherein the catalyst is used in chemical processes at the petrochemical plant; and
    continuously predicting a selectivity of the catalyst under different loads and in real-time using a trained machine learning model, wherein the actual operational data, the real-time external data, are inputs to the trained machine learning model, and wherein the trained machine learning model is trained using an actual selectivity and historical catalyst performance.

12. The method of claim 11, further comprising: determining, at least in part, an optimum time to replace the catalyst at the petrochemical plant using the predicted selectivity.

13. The method of claim 12, wherein the optimum time to replace the catalyst at the petrochemical plant is determined using a physics-based model for the catalyst.

14. An apparatus comprising a non-transitory, computer readable, storage medium that stores instructions that, when executed by at least one processor, cause the at least one processor to perform operations comprising:

   capturing real-time external data associated with a catalyst;
   obtaining real-time operational data associated with a petrochemical plant, wherein the catalyst is used in chemical processes at the petrochemical plant; and
   continuously predicting a selectivity of the catalyst under different loads and in real-time using a trained machine learning model, wherein the actual operational data, the real-time external data, are inputs to the trained machine learning model, and wherein the trained machine learning model is trained using an actual selectivity and historical catalyst performance.

15. The apparatus of claim 14, wherein the operations comprise determining an optimum time to replace the catalyst at the petrochemical plant using the predicted selectivity.

# Fig. 1A

# Fig. 1B

# Fig. 2

200

214

**212** Actual Selectivity → **214** Variable Cost → **218** The Gain →

**216** Fixed Cost (Catalyst & Change out) ↑ Variable Cost

210

220 — Catalyst Optimum Cycle Model

**222** Calculating the Gain at Multiple Scenarios (Run Lengths) → **224** Calculating all resulted gains and pick up the best scenario and its corresponding selectivity

234 — Finding out the run length and change out date of the optimum selectivity

**232** For Each Run Length, selectivity is calculated

230 — Catalyst Selectivity Forecast Model

EP 4 730 236 A1

13

# Fig. 3A

300A

# Fig. 3B

300B

# Fig. 4

400

Processor
410

Memory
420

450

440

Storage Device
430

Input/Output

Input/Output Device
460

Europäisches
Patentamt

European
Patent Office

Office européen
des brevets

# EUROPEAN SEARCH REPORT

**Application Number**

EP 24 20 6857

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/150307 A1 (WELLS GARY JAMES [US] ET AL) 9 May 2024 (2024-05-09) * the whole document * ----- | 1-15 | INV. G06Q10/0639 G05B23/02 G06N20/00 G16C20/70 |
| A | US 2024/104464 A1 (HONG UNG GI [KR] ET AL) 28 March 2024 (2024-03-28) * abstract * ----- | 1-15 | |
| A | US 2024/095548 A1 (HONG UNG GI [KR] ET AL) 21 March 2024 (2024-03-21) * abstract * ----- | 1-15 | |

**TECHNICAL FIELDS SEARCHED (IPC)**

G06Q
G06N
G16C
G05B

The present search report has been drawn up for all claims

| Place of search | Date of completion of the search | Examiner |
|---|---|---|
| Munich | 1 April 2025 | Weidmann, Matthias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

....................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 20 6857

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2025

| Patent document cited in search report | | Publication date | Patent family member(s) | | Publication date |
|---|---|---|---|---|---|
| US 2024150307 | A1 | 09-05-2024 | BR | 112023020953 A2 | 06-02-2024 |
| | | | CA | 3214971 A1 | 13-10-2022 |
| | | | CN | 117099163 A | 21-11-2023 |
| | | | EP | 4320621 A1 | 14-02-2024 |
| | | | JP | 2024514571 A | 02-04-2024 |
| | | | KR | 20230167759 A | 11-12-2023 |
| | | | TW | 202239751 A | 16-10-2022 |
| | | | US | 2024150307 A1 | 09-05-2024 |
| | | | WO | 2022214539 A1 | 13-10-2022 |
| US 2024104464 | A1 | 28-03-2024 | EP | 4273646 A1 | 08-11-2023 |
| | | | KR | 20220147932 A | 04-11-2022 |
| | | | US | 2024104464 A1 | 28-03-2024 |
| | | | WO | 2022231282 A1 | 03-11-2022 |
| US 2024095548 | A1 | 21-03-2024 | EP | 4273652 A1 | 08-11-2023 |
| | | | KR | 20220147936 A | 04-11-2022 |
| | | | US | 2024095548 A1 | 21-03-2024 |
| | | | WO | 2022231283 A1 | 03-11-2022 |

EPO FORM P0459

For more details about this annex : see Official Journal of the European Patent Office, No. 12/82